# EUROPEAN PATENT APPLICATION

(11) **EP 4 509 487 A1**
(43) Date of publication of application: **19.02.2025**
(21) Application number: 23788358.2
(22) Date of filing: 12.04.2023
(51) Int. Cl.: C07B 33/00, C11D 17/08, C11D 3/28, C11D 7/32, C11D 7/54, C09K 3/00

(54) **LIQUID COMPOSITION**

(30) Priority: 13.04.2022 JP 2022066552
(71) Applicant: Kao Corporation, Tokyo 103-8210 (JP)
(72) Inventor: INAGAKI Kensuke, Wakayama-shi, Wakayama 640-8580 (JP)
(74) Representative: Hoffmann Eitle
(86) International application number: PCT/JP2023/014847
(87) International publication number: WO 2023/199940

(57) **Abstract**

The present invention is a liquid composition containing (A) an isoxazolium salt and water, the composition being acidic.

## Description

### Field of the Invention

The present invention relates to a liquid composition which can be utilized as an oxidizing agent, a bleaching agent or the like.

### Background of the Invention

It is known that, of cationic compounds, isoxazolium salts can be utilized as condensing agents or the like. An oxidizing agent exhibits an oxidizing effect on other substances, and on colored substances, the oxidizing effect can be recognized as a phenomenon such as decolorization, bleaching, discoloration or the like. Oxidizing agents are used for various applications such as bleaching of hard articles in bathrooms, toilets, kitchens and others, mold removal, bleaching of clothing, decomposition of substances causative of stains or odor, disinfection, bleaching of pulp, scouring of fibers and others.

On the other hand, as bleaching agents, in addition to so-called chlorine bleaches using hypochlorite salts and others, oxygen bleaches using peroxy acids such as hydrogen peroxide, borates, percarbonates, persilicates, superphosphates and others are known. In oxygen bleaches, bleach activators such as organic acid esters and others are often used together to increase their bleaching power. In oxygen bleaches, activated bleaching species (organic peroxy acids or the like) are produced by reacting oxidizing agents (for example, hydrogen peroxide) with organic acid esters or the like as bleach activators, and objects are oxidized and bleached therewith.

JP-A H11-256188 discloses a detergent or a cleaner containing a peroxy compound and a specific formamidinium salt in amounts falling within their respective specific ranges.

### Summary of the Invention

The present invention provides a liquid composition containing an isoxazolium salt and excellent in storage stability.

The present invention relates to a liquid composition containing (A) an isoxazolium salt [hereinafter referred to as component (A)] and water, the composition being acidic.

Further, the present invention relates to a method for storing an isoxazolium salt including, storing component (A) in an acidic solution.

Further, the present invention relates to an oxidizing method including, bringing a treatment liquid prepared from the liquid composition of the present invention into contact with an object in the presence of hydrogen peroxide.

Further, the present invention relates to a bleaching method including, bringing a treatment liquid prepared from the liquid composition of the present invention into contact with an object in the presence of hydrogen peroxide.

Further, the present invention relates to use of the liquid composition of the present invention for the production of oxidizing agents.

Further, the present invention relates to use of the liquid composition of the present invention for the production of bleaching agents.

According to the present invention, provided is a liquid composition containing a compound selected from isoxazolium salts and excellent in storage stability.

### Embodiments of the Invention

### [Liquid composition]

The liquid composition of the present invention contains an isoxazolium salt of component (A) and water, the composition being acidic.

Examples of the isoxazolium salt of component (A) include cationized compounds of compounds having isoxazole skeletons. Specific examples of component (A) include, for example, a compound of the following general formula: 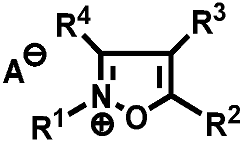 wherein A⁻ represents an anion, R¹ and R² each independently represent an alkyl group with 1 or more and 24 or less carbons which may contain a heteroatom, R³ and R⁴ each independently represent a group selected from a hydrogen atom, an alkyl group with 1 or more and 24 or less carbons which may contain a heteroatom, -COOR⁵ and - CONHR⁶, and R⁵ and R⁶ each represent an alkyl group with 2 or more and 23 or less carbons.

In the above formula, the anion of A⁻ may be, for example, either an organic anion or an inorganic anion. Examples of the anion of A⁻ include, for example, an anion which is a conjugate base of an acid with a pKa of 5 or less. The anion of A⁻ is preferably an anion other than an iodide ion. Examples of the anion of A⁻ include, for example, sulfonic acid ions such as a trifluoromethanesulfonate ion (⁻OTf), a para toluenesulfonate ion (⁻OTs), a methanesulfonate ion (⁻OMs) and others, alkyl sulfate ions such as a methyl sulfate ion (⁻OSO₃Me) and others, halide ions such as a chloride ion (Cl⁻), a bromide ion (Br⁻) and others (excluding an iodide ion (I⁻)), fluoroborate ions such as a tetrafluoroborate ion (BF₄⁻) and others, fluorophosphate ions such as a hexafluorophosphate ion (PF₆⁻) and others, imide ions such as a bis(trifluoromethanesulfonyl) imide ion ((CF₃SO₂)₂N⁻) and others, fluoroacetate ions such as a trifluoroacetate ion (CF₃COO⁻) and others, a perchlorate ion (ClO₄⁻), a chlorate ion (ClO₃⁻), a chlorite ion (ClO₂⁻), and others. A⁻ is preferably an anion selected from ⁻OTf, ⁻OSO₃Me,⁻OTs, ⁻OMs, Cl⁻ and Br⁻.

In the above formula, R¹ and R² each independently represent an alkyl group with 1 or more and 24 or less carbons which may contain a heteroatom. Examples of the heteroatom include, for example, a nitrogen atom, an oxygen atom, a sulfur atom and a phosphorus atom. When R¹ or R² is an alkyl group containing a heteroatom, the number of carbons in R¹ or R² may be a total number of carbons in the carbon chain excluding the heteroatom.

The alkyl group of R¹ is preferably a linear alkyl group from the viewpoint of improving oxidative decomposition activity, and preferably a branched alkyl group from the viewpoint of excellent water solubility.

The alkyl group of R¹ has one or more carbons, and may have, for example, 2 or more, further 3 or more and further 4 or more carbons, and has 24 or less carbons, and may have, for example, 20 or less, further 16 or less and further 12 or less carbons.

The alkyl group of R² may be linear or branched, and is preferably a linear alkyl group from the viewpoint of improving oxidative decomposition activity.

The alkyl group of R² has 1 or more and 24 or less carbons, and may have, for example, 20 or less, further 16 or less, further 12 or less, further 8 or less, further 4 or less and further 2 or less carbons.

In the above formula, R³ and R⁴ each independently represent a group selected from a hydrogen atom, an alkyl group with 1 or more and 24 or less carbons which may contain a heteroatom, -COOR⁵ and -CONHR⁶, and R⁵ and R⁶ each represent an alkyl group with 2 or more and 23 or less carbons. Examples of the heteroatom are the same as those stated in R¹ and R².

R³ is preferably a group selected from a hydrogen atom, an alkyl group (an alkyl group not containing a heteroatom), -COOR⁵ and -CONHR⁶ from the viewpoint of availability. R⁵ and R⁶ each represent an alkyl group with 2 or more and 23 or less carbons.

When R³ is an alkyl group, R³ may be a linear alkyl group or a branched alkyl group, and the alkyl group of R³ has 1 or more and 24 or less carbons, and may have, for example, 20 or less, further 16 or less, further 12 or less, further 8 or less, further 4 or less and further 2 or less carbons.

R⁵ and R⁶ may each be a linear alkyl group or a branched alkyl group, and the alkyl groups of R⁵ and R⁶ may each have, for example, 4 or more and 12 or less carbons.

R⁴ is preferably a hydrogen atom or an alkyl group with 1 or more and 4 or less carbons and more preferably a hydrogen atom from the viewpoint of availability.

The liquid composition of the present invention preferably contains component (A) in an amount of, for example, 0.01 mass% or more, further 0.1 mass% or more and further 1 mass% or more, and 20 mass% or less, further 10 mass% or less and further 5 mass% or less from the viewpoint of improving oxidative decomposition activity.

The liquid composition of the present invention contains water. Examples of the water include ion-exchanged water, tap water and others. Water can be used in an amount for making a total of the makeup of the liquid composition of the present invention 100 mass%. The liquid composition of the present invention can contain water in an amount of, for example, 50 mass% or more, further 80 mass% or more and further 90 mass% or more, and 99 mass% or less, further 95 mass% or less and further 90 mass% or less.

The liquid composition of the present invention can contain (B) hydrogen peroxide [hereinafter referred to as component (B)]. When the liquid composition of the present invention contains component (B), the composition preferably contains component (B) in an amount of, for example, 0.01 mass% or more, further 1 mass% or more and further 3 mass% or more, and 30 mass% or less, further 20 mass% or less and further 10 mass% or less from the viewpoint of being excellent in safety.

When the liquid composition of the present invention contains component (B), a content of component (B) relative to a content of 1 part by mole of component (A) is preferably, for example, 1 part by mole or more, further 22 parts by mole or more and further 33 parts by mole or more, and 100 parts by mole or less, further 55 parts by mole or less and further 44 parts by mole or less from the viewpoint of improving oxidative decomposition activity.

The liquid composition of the present invention can contain optional components other than component (B). Examples of such optional components include, for example, chelating agents, thickeners, surfactants, polymers and others. Note that component (B) may be combined as a hydrogen peroxide solution. When a predetermined component is combined as an aspect containing water like a hydrogen peroxide solution, such water forms part or all of a water component in the liquid composition of the present invention.

The liquid composition of the present invention is suitable for use in oxidizing agents. In other words, for example, the liquid composition of the present invention can be used as an oxidizing agent together with hydrogen peroxide, or used for the production of oxidizing agents.

Further, the liquid composition of the present invention is suitable for use in bleaching agents. In other words, for example, the liquid composition of the present invention can be used as a bleaching agent together with hydrogen peroxide, or used for the production of bleaching agents.

A concentration of component (A) when the liquid composition of the present invention is used, for example, when the liquid composition is used as an oxidizing agent or a bleaching agent may be, for example, 0.0001 mass% or more, further 0.001 mass% or more and further 0.01 mass% or more, and 10 mass% or less, further 1 mass% or less and further 0.1 mass% or less.

Further, when the liquid composition of the present invention contains component (B), a concentration of component (B) when the liquid composition is used, for example, when the liquid composition is used as an oxidizing agent or a bleaching agent may be, for example, 0.001 mass% or more, further 0.01 mass% or more and further 0.1 mass% or more, and 30 mass% or less, further 3 mass% or less and further 1 mass% or less.

The liquid composition of the present invention can also be obtained, for example, by mixing a solid agent containing component (A) with water. In other words, the present invention provides a kit for an acidic liquid composition containing component (A) and water, the kit containing a solid agent containing component (A).

The liquid composition of the present invention may have a pH of, for example, 2 or more and further 3 or more, and less than 7, further 6 or less and further 5 or less at 25°C. The liquid composition can contain, for example, a pH adjuster and other components to have such a pH. Examples of the pH adjuster include hydrogen phosphates such as disodium hydrogen phosphate, potassium dihydrogen phosphate and others, organic acids such as lactic acid, succinic acid, gluconic acid, citric acid, acetic acid, tartaric acid and others, inorganic acids such as phosphoric acid, boric acid, hydrochloric acid, sulfuric acid and others, sulfonic acids such as para toluenesulfonic acid, camphorsulfonic acid, methanesulfonic acid and others, or the like. pH adjusters may be selected in combination to have a buffer capacity.

The liquid composition of the present invention can be used for various applications such as bleaching of hard articles in bathrooms, toilets, kitchens and others, mold removal, bleaching of clothing, decomposition of substances causative of stains or odor, disinfection, virus killing, bleaching of pulp and others where oxidizing action produces useful effects on the treated objects. The liquid composition of the present invention can be used as, for example, a bleaching agent composition, a bleaching cleaning agent composition, a mold removing agent composition, a decolorizing agent composition, a deodorizing agent composition, a sterilizing agent composition, a virus killing agent composition or the like in combination with hydrogen peroxide or the like as necessary. Especially, the liquid composition of the present invention is preferably used for bleaching or bleaching cleaning. Further, the liquid composition is preferably used for bleaching or bleaching cleaning of hard articles.

The present invention provides use of the liquid composition of the present invention for the production of oxidizing agents.

Further, the present invention provides use of the liquid composition of the present invention for the production of bleaching agents.

If the liquid composition of the present invention is of composition suitable for oxidation or bleaching, it is also possible to use the composition as-is to produce an oxidizing agent or a bleaching agent.

In the above use, the oxidizing agents or bleaching agents preferably contain component (A) in an amount of, for example, 0.0001 mass% or more, further 0.001 mass% or more and further 0.01 mass% or more, and 10 mass% or less, further 1 mass% or less and further 0.1 mass% or less from the viewpoint of improving oxidative decomposition activity. The composition can contain component (A) in an amount falling within this range.

In the above use, the oxidizing agents or bleaching agents preferably contain component (B). In that case, the oxidizing agents or bleaching agents preferably contain component (B) in an amount of, for example, 0.001 mass% or more, further 0.01 mass% or more and further 0.1 mass% or more, and 30 mass% or less, further 3 mass% or less and further 1 mass% or less. The composition can contain component (B) in an amount falling within this range.

### [Oxidizing method and bleaching method]

The present invention provides an oxidizing method including, bringing a treatment liquid prepared from the liquid composition of the present invention into contact with an object in the presence of hydrogen peroxide.

Further, the present invention provides a bleaching method including, bringing the treatment liquid prepared from the liquid composition of the present invention (hereinafter also referred to as the treatment liquid of the present invention) into contact with an object in the presence of hydrogen peroxide.

The treatment liquid of the present invention is preferably a treatment liquid containing components (A) and (B) and water. If the liquid composition of the present invention is of composition suitable for oxidation or bleaching, it is also possible to use the composition as-is as the treatment liquid of the present invention. Further, the treatment liquid of the present invention may be prepared by mixing a composition containing component (A) and not containing component (B) with a composition containing a compound which is a supply source for component (B) or component (B).

The treatment liquid of the present invention can be obtained by mixing the liquid composition of the present invention containing component (A) and water with component (B), and/or a supply source for component (B), for example, a peroxide which produces hydrogen peroxide in water, and as necessary, water. The peroxide which produces hydrogen peroxide in water is exemplified by an inorganic peroxide or a hydrogen peroxide adduct, and is preferably a percarbonate, a tripolyphosphate/hydrogen peroxide adduct, a pyrophosphate/hydrogen peroxide adduct, urea/hydrogen peroxide adduct, a sulfate/hydrogen peroxide adduct, a perborate, a persilicate or a peroxide salt and more preferably sodium percarbonate, sodium tripolyphosphate/hydrogen peroxide adduct, sodium pyrophosphate/hydrogen peroxide adduct, urea/hydrogen peroxide adduct, or 4Na₂SO₄/2E₂O₂, sodium perborate monohydrate, sodium perborate tetrahydrate, sodium persilicate, sodium peroxide, calcium peroxide or the like. Further, the treatment liquid can also be obtained by preparing the liquid composition of the present invention using a kit as described above, and thereafter mixing the composition with component (B) and/or a supply source for component (B), and as necessary, water. Further, as described later, depending on the use purpose, a pH adjuster, for example, one also functioning as a buffering agent like a phosphate buffer solution may be added to adjust a pH of the treatment liquid to, for example, 3 or more, further 5 or more and further 6 or more, and 12 or less, further 10 or less and further 8 or less.

The treatment liquid of the present invention preferably contains component (A) in an amount of, for example, 0.0001 mass% or more, further 0.001 mass% or more and further 0.01 mass% or more, and 10 mass% or less, further 1 mass% or less and further 0.1 mass% or less from the viewpoint of improving oxidative decomposition activity.

The treatment liquid of the present invention can contain component (B) in an amount of, for example, 0.001 mass% or more, further 0.01 mass% or more and further 0.1 mass% or more, and 30 mass% or less, further 3 mass% or less and further 1 mass% or less.

The treatment liquid of the present invention can contain water in an amount of 80 mass% or more, further 90 mass% or more and further 95 mass% or more, and 99.99 mass% or less, further 98 mass% or less and further 95 mass% or less.

Examples of the object in the oxidizing method and the bleaching method include, for example, hard articles in bathrooms, toilets, kitchens and others, textile products such as clothing and others, or the like. In the oxidizing method and the bleaching method, the liquid composition of the present invention can be brought into contact with the object in the presence of hydrogen peroxide by a method such as immersing, applying, spraying or the like. A contact time or a contact temperature when the treatment liquid of the present invention is brought into contact with the object is not limited.

The treatment liquid of the present invention can contain the optional components stated in the liquid composition of the present invention.

The treatment liquid of the present invention may have a pH of, for example, 3 or more, further 5 or more and further 6 or more, and 12 or less, further 10 or less and further 8 or less. This pH is a pH at a temperature at which the oxidizing method or the bleaching method of the present invention is carried out, and may be, for example, a pH at 25°C. In the oxidizing method or the bleaching method of the present invention, excellent oxidizing power or bleaching power can be obtained even at a pH near neutral in the presence of hydrogen peroxide.

### [Storing method]

The present invention provides a method for storing a compound including, storing component (A) in an acidic solution (hereinafter also referred to as the storing method of the present invention).

The matters stated in the liquid composition of the present invention can be appropriately applied to the storing method of the present invention. Specific examples or preferable examples of component (A) and others in the storing method of the present invention are also the same as in the liquid composition of the present invention. The storing method of the present invention may be a method for storing component (A) or the liquid composition of the present invention.

In the storing method of the present invention, a content of component (A) in the acidic solution may be, for example, 0.01 mass% or more, further 0.1 mass% or more and further 1 mass% or more, and 20 mass% or less, 10 mass% or less and further 5 mass% or less.

In the storing method of the present invention, component (A) is stored in the acidic solution. The acidic solution used for storage may have a pH of, for example, 2 or more and further 3 or more, and less than 7, further 6 or less and further 5 or less. The acidic solution can contain, for example, a pH adjuster and other components to have such a pH. Examples of the pH adjuster include hydrogen phosphates such as disodium hydrogen phosphate, potassium dihydrogen phosphate and others, organic acids such as lactic acid, succinic acid, gluconic acid, citric acid, acetic acid, tartaric acid and others, inorganic acids such as phosphoric acid, boric acid, hydrochloric acid, sulfuric acid and others, sulfonic acids such as para toluenesulfonic acid, camphorsulfonic acid, methanesulfonic acid and others, or the like. Note that this pH is a pH in a state where component (A) and optional components such as component (B) and others are contained.

The storing method of the present invention can be carried out under the coexistence of components other than component (A). For example, storing can be carried out in the acidic solution with the optional components stated in the liquid composition of the present invention added thereto.

In the present invention, component (A) is preferably stored in the acidic solution under the coexistence of hydrogen peroxide of component (B) from the viewpoint of excellent handleability. When component (B) is made to coexist in the storing method of the present invention, a content of component (B) in the acidic solution may be, for example, 0.01 mass% or more, further 1 mass% or more and further 3 mass% or more, and 30 mass% or less, further 20 mass% or less and further 10 mass% or less.

The storing method of the present invention can be used as a method for storing, for example, the liquid composition of the present invention, and further, the liquid composition of the present invention used for oxidizing agents or the liquid composition of the present invention used for bleaching agents.

In the storing method of the present invention, a temperature of the acidic solution is not particularly limited, but may be, for example, 0°C or more and further 25°C or more, and 60°C or less and further 50°C or less.

In the storing method of the present invention, a storage period is not particularly limited, but may be, for example, 1 day or more and further 14 days or more, and 730 days or less and further 365 days or less.

In the storing method of the present invention, component (A) can be stored in such a manner that, for example, water and component (A), and as necessary, component (B) and other optional components are put into a predetermined container, and the solution is adjusted to be acidic in nature and preferably adjusted to have a pH falling within the above range. A shape, capacity or the like of the container is not limited. The container preferably has a sealing member for an opening, such as a lid, a stopper or the like.

In addition to the aforementioned embodiments, the present invention discloses the aspects below.
<1> A liquid composition containing (A) an isoxazolium salt [hereinafter referred to as component (A)] and water, the composition being acidic.
<2> The liquid composition according to <1>, wherein component (A) is a compound represented by the following general formula: 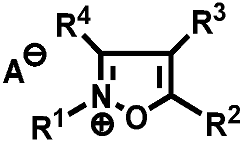 wherein A⁻ represents an anion, R¹ and R² each independently represent an alkyl group with 1 or more and 24 or less carbons which may contain a heteroatom, R³ and R⁴ each independently represent a group selected from a hydrogen atom, an alkyl group with 1 or more and 24 or less carbons which may contain a heteroatom, -COOR⁵ and - CONHR⁶, and R⁵ and R⁶ each represent an alkyl group with 2 or more and 23 or less carbons.
<3> The liquid composition according to <2>, wherein in the formula, A⁻ is an anion selected from an organic anion and an inorganic anion.
<4> The liquid composition according to <2> or <3>, wherein in the formula, A⁻ is an anion which is a conjugate base of an acid with a pKa of 5 or less.
<5> The liquid composition according to any of <2> to <4>, wherein in the formula, A⁻ is an anion selected from sulfonic acid ions, alkyl sulfate ions, halide ions (excluding an iodide ion (I⁻)), fluoroborate ions, fluorophosphate ions, imide ions, fluoroacetate ions, a perchlorate ion (ClO₄⁻) , a chlorate ion (ClO₃⁻) and a chlorite ion (ClO₂⁻), and further, an anion selected from a trifluoromethanesulfonate ion (⁻OTf), a para toluenesulfonate ion (⁻OTs), a methanesulfonate ion (⁻OMs), a methyl sulfate ion (⁻OSO₃Me), a chloride ion (Cl⁻), a bromide ion (Br⁻), a tetrafluoroborate ion (BF₄⁻), a hexafluorophosphate ion (PF₆⁻), a bis(trifluoromethanesulfonyl) imide ion ((CF₃SO₂)₂N⁻), a trifluoroacetate ion (CF₃COO⁻), a perchlorate ion (ClO₄⁻), a chlorate ion (ClO₃⁻) and a chlorite ion (ClO₂⁻).
<6> The liquid composition according to any of <2> to <5>, wherein in the formula, A⁻ is an anion selected from ⁻OTf, ⁻OSO₃Me, ⁻OTs, ⁻OMs, Cl⁻, Br⁻ and ClO₄⁻.
<7> The liquid composition according to any of <2> <6>, wherein in the formula, R¹ and R² each independently represent an alkyl group with 1 or more and 24 or less carbons which may contain a heteroatom, and the heteroatom is at least one selected from a nitrogen atom, an oxygen atom, a sulfur atom and a phosphorus atom.
<8> The liquid composition according to any of <2> to <7>, wherein in the formula, the alkyl group of R¹ is a linear alkyl group.
<9> The liquid composition according to any of <2> to <7>, wherein in the formula, the alkyl group of R¹ is a branched alkyl group.
<10> The liquid composition according to any of <2> to <9>, wherein in the formula, the alkyl group of R¹ has 1 or more, further 2 or more, further 3 or more and further 4 or more, and 24 or less, further 20 or less, further 16 or less and further 12 or less carbons.
<11> The liquid composition according to any of <2> to <10>, wherein in the formula, the alkyl group of R² is a linear or branched alkyl group and further a linear alkyl group.
<12> The liquid composition according to any of <2> to <11>, wherein in the formula, the alkyl group of R² has 20 or less, further 16 or less, further 12 or less, further 8 or less, further 4 or less and further 2 or less carbons.
<13> The liquid composition according to any of <2> to <12>, wherein in the formula, R³ and R⁴ each independently represent a group selected from a hydrogen atom, an alkyl group with 1 or more and 24 or less carbons which may contain a heteroatom, -COOR⁵ and - CONHR⁶, R⁵ and R⁶ each represent an alkyl group with 2 or more and 23 or less carbons, and the heteroatom is at least one selected from a nitrogen atom, an oxygen atom, a sulfur atom and a phosphorus atom.
<14> The liquid composition according to any of <2> to <13>, wherein in the formula, R³ is a group selected from a hydrogen atom, an alkyl group (an alkyl group not containing a heteroatom), -COOR⁵ and -CONHR⁶, and R⁵ and R⁶ each represent an alkyl group with 2 or more and 23 or less carbons.
<15> The liquid composition according to any of <2> to <14>, wherein in the formula, when R³ is an alkyl group, R³ may be a linear alkyl group or a branched alkyl group, and the alkyl group of R³ has 1 or more and 24 or less carbons and further 20 or less, further 16 or less, further 12 or less, further 8 or less, further 4 or less and further 2 or less carbons.
<16> The liquid composition according to any of <2> to <15>, wherein in the formula, R⁵ and R⁶ each represent a linear alkyl group or a branched alkyl group, and the alkyl groups of R⁵ and R⁶ each have 4 or more and 12 or less carbons.
<17> The liquid composition according to any of <2> to <16>, wherein in the formula, R⁴ is a hydrogen atom or an alkyl group with 1 or more and 4 or less carbons and further a hydrogen atom.
<18> The liquid composition according to any of <2> to <17>, wherein in the formula, A⁻ is ⁻OTf, R¹ and R² each represent a methyl group, R³ is a group selected from - COOR⁵ and -CONHR⁶, R⁵ and R⁶ each represent a butyl group or a dodecyl group, and R⁴ is a hydrogen atom.
<19> The liquid composition according to any of <2> to <18>, wherein in the formula, A⁻ is ⁻OTf, R¹ and R² each represent a methyl group, R³ is -COOR⁵, R⁵ is a butyl group, and R⁴ is a hydrogen atom.
<20> The liquid composition according to any of <2> to <18>, wherein in the formula, A⁻ is ⁻OTf, R¹ and R² each represent a methyl group, R³ is -COOR⁵, R⁵ is a dodecyl group, and R⁴ is a hydrogen atom.
<21> The liquid composition according to any of <2> to <18>, wherein in the formula, A⁻ is ⁻OTf, R¹ and R² each represent a methyl group, R³ is -CONHR⁶, R⁶ is a dodecyl group, and R⁴ is a hydrogen atom.
<22> The liquid composition according to any of <2> to <17>, wherein in the formula, A⁻ is ⁻ClO₄⁻, R¹ is a tert-butyl group, R² is a methyl group, and R³ and R⁴ each represent a hydrogen atom.
<23> The liquid composition according to any of <1> to <22>, containing component (A) in an amount of 0.01 mass% or more, further 0.1 mass% or more and further 1 mass% or more, and 20 mass% or less, further 10 mass% or less and further 5 mass% or less.
<24> The liquid composition according to any of <1> to <23>, containing water in an amount of 50 mass% or more, further 80 mass% or more and further 90 mass% or more, and 99 mass% or less, further 95 mass% or less and further 90 mass% or less.
<25> The liquid composition according to any of <1> to <24>, further containing (B) hydrogen peroxide [hereinafter referred to as component (B)].
<26> The liquid composition according to <25>, containing component (B) in an amount of 0.01 mass% or more, further 1 mass% or more and further 3 mass% or more, and 30 mass% or less, further 20 mass% or less and further 10 mass% or less.
<27> The liquid composition according to <25> or <26>, wherein a content of component (A) relative to a content of 1 part by mole of component (B) is 1 part by mole or more, further 22 parts by mole or more and further 33 parts by mole or more, and 100 parts by mole or less, further 55 parts by mole or less and further 44 parts by mole or less.
<28> The liquid composition according to any of <25> to <27>, wherein a concentration of component (B) when the liquid composition is used is 0.001 mass% or more, further 0.01 mass% or more and further 0.1 mass% or more, and 30 mass% or less, further 3 mass% or less and further 1 mass% or less.
<29> The liquid composition according to any of <1> to <28>, wherein the liquid composition is used for oxidizing agents.
<30> The liquid composition according to any of <1> to <29>, wherein the liquid composition is used for bleaching agents.
<31> The liquid composition according to any of <1> to <30>, wherein a concentration of component (A) when the liquid composition is used is 0.0001 mass% or more, further 0.001 mass% or more and further 0.01 mass% or more, and 10 mass% or less, further 1 mass% or less and further 0.1 mass% or less.
<32> The liquid composition according to any of <1> to <31>, wherein the liquid composition has a pH of 2 or more and further 3 or more, and less than 7, further 6 or less and further 5 or less at 25°C.
<33> The liquid composition according to any of <1> to <32>, containing a pH adjuster.
<34> The liquid composition according to <33>, wherein the pH adjuster is a pH adjuster selected from a hydrogen phosphate selected from disodium hydrogen phosphate and potassium dihydrogen phosphate, an organic acid selected from lactic acid, succinic acid, gluconic acid, citric acid, acetic acid and tartaric acid, an inorganic acid selected from phosphoric acid, boric acid, hydrochloric acid and sulfuric acid, and a sulfonic acid selected from para toluenesulfonic acid, camphorsulfonic acid and methanesulfonic acid.
<35> An oxidizing method including, bringing a treatment liquid prepared from the liquid composition according to any of <1> to <34> into contact with an object in the presence of hydrogen peroxide.
<36> A bleaching method including, bringing a treatment liquid prepared from the liquid composition according to any of <1> to <34> into contact with an object in the presence of hydrogen peroxide.
<37> The method according to <35> or <36>, wherein the treatment liquid contains components (A) and (B) and water.
<38> The method according to <37>, wherein the treatment liquid contains component (B) in an amount of 0.001 mass% or more, further 0.01 mass% or more and further 0.1 mass% or more, and 30 mass% or less, further 3 mass% or less and further 1 mass% or less.
<39> The method according to any of <35> to <38>, wherein the treatment liquid contains component (A) in an amount of 0.0001 mass% or more, further 0.001 mass% or more and further 0.01 mass% or more, and 10 mass% or less, further 1 mass% or less and further 0.1 mass% or less.
<40> The method according to any of <35> to <39>, wherein the treatment liquid contains water in an amount of 80 mass% or more, further 90 mass% or more and further 95 mass% or more, and 99.99 mass% or less, further 98 mass% or less and further 95 mass% or less.
<41> The method according to any of <35> to <40>, wherein the treatment liquid has a pH of 3 or more, further 5 or more and further 6 or more, and 12 or less, further 10 or less and further 8 or less.
<42> A method for storing an isoxazolium salt including, storing (A) an isoxazolium salt [hereinafter referred to as component (A)] in an acidic solution.
<43> The method for storing an isoxazolium salt according to <42>, wherein a content of component (A) in the solution is 0.01 mass% or more, further 0.1 mass% or more and further 1 mass% or more, and 20 mass% or less, 10 mass% or less and further 5 mass% or less.
<44> The method for storing an isoxazolium salt according to <42> or <43>, wherein component (A) is stored in a solution having a pH of 2 or more and further 3 or more, and less than 7, further 6 or less and further 5 or less.
<45> The method for storing an isoxazolium salt according to any of <42> to <44>, wherein the solution in which component (A) is stored contains a pH adjuster.
<46> The method for storing an isoxazolium salt according to <45>, wherein the pH adjuster is a pH adjuster selected from a hydrogen phosphate selected from disodium hydrogen phosphate and potassium dihydrogen phosphate, an organic acid selected from lactic acid, succinic acid, gluconic acid, citric acid, acetic acid and tartaric acid, an inorganic acid selected from phosphoric acid, boric acid, hydrochloric acid and sulfuric acid, and a sulfonic acid selected from para toluenesulfonic acid, camphorsulfonic acid and methanesulfonic acid.
<47> The method for storing an isoxazolium salt according to any of <42> to <46>, wherein component (A) is stored under the coexistence of (B) hydrogen peroxide [hereinafter referred to as component (B)].
<48> The method for storing an isoxazolium salt according to any of <42> to <47>, wherein a content of component (B) in the solution is 0.01 mass% or more, further 1 mass% or more and further 3 mass% or more, and 30 mass% or less, further 20 mass% or less and further 10 mass% or less.
<49> The storing method according to any of <42> to <48>, wherein the storing method is a method for storing the liquid composition according to any of <1> to <34>, and further the liquid composition used for oxidizing agents or the liquid composition used for bleaching agents.
<50> The storing method according to any of <42> to <49>, wherein a temperature of the acidic solution is 0°C or more and further 25°C or more, and 60°C or less and further 50°C or less.
<51> The storing method according to any of <42> to <50>, wherein a storage period is 1 day or more and further 14 days or more, and 730 days or less and further 365 days or less.
<52> Use of the liquid composition according to any of <1> to <34> for the production of oxidizing agents.
<53> Use of the liquid composition according to any of <1> to <34> for the production of bleaching agents.
<54> A liquid composition containing (A) an isoxazolium salt represented by the following general formula in an amount of 0.01 mass% or more and 5 mass% or less, (B) hydrogen peroxide in an amount of 0.01 mass% or more and 10 mass% or less, and water in an amount of 80 mass% or more and 99 mass% or less, the composition being acidic, 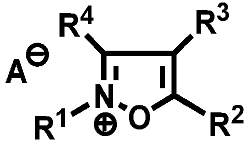 wherein A⁻ is ⁻OTf, R¹ and R² each represent a methyl group, R³ is -COOR⁵, R⁵ is a butyl group, and R⁴ is a hydrogen atom.
<55> A liquid composition containing (A) an isoxazolium salt represented by the following general formula in an amount of 0.01 mass% or more and 5 mass% or less, (B) hydrogen peroxide in an amount of 0.01 mass% or more and 10 mass% or less, and water in an amount of 80 mass% or more and 99 mass% or less, the composition being acidic, 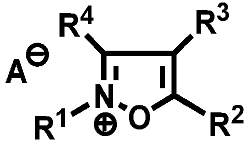 wherein A⁻ is ⁻OTf, R¹ and R² each represent a methyl group, R³ is -COOR⁵, R⁵ is a dodecyl group, and R⁴ is a hydrogen atom.
<56> A liquid composition containing (A) an isoxazolium salt represented by the following general formula in an amount of 0.01 mass% or more and 5 mass% or less, (B) hydrogen peroxide in an amount of 0.01 mass% or more and 10 mass% or less, and water in an amount of 80 mass% or more and 99 mass% or less, the composition being acidic, 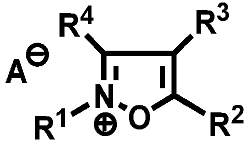 wherein A⁻ is ⁻OTf, R¹ and R² each represent a methyl group, R³ is -CONHR⁶, R⁶ is a dodecyl group, and R⁴ is a hydrogen atom.
<57> A liquid composition containing (A) an isoxazolium salt represented by the following general formula in an amount of 0.01 mass% or more and 5 mass% or less, (B) hydrogen peroxide in an amount of 0.01 mass% or more and 10 mass% or less, and water in an amount of 80 mass% or more and 99 mass% or less, the composition being acidic, 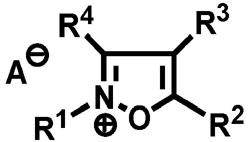 wherein A⁻ is ClO₄⁻, R¹ is a tert-butyl group, R² is a methyl group, and R³ and R⁴ each represent a hydrogen atom.
<58> An oxidizing method or a bleaching method including, bringing a treatment liquid into contact with an object, the treatment liquid being prepared from a liquid composition containing (A) an isoxazolium salt represented by the following general formula [hereinafter referred to as component (A)], (B) hydrogen peroxide [hereinafter referred as component (B)] and water, the liquid composition being acidic,
   wherein the treatment liquid has a pH of 5 or more and 10 or less,
   the treatment liquid contains component (A) in an amount of 0.0001 mass% or more and 1 mass% or less,
   the treatment liquid contains component (B) in an amount of 0.001 mass% or more and 3 mass% or less, and
   the treatment liquid contains water in an amount of 80 mass% or more and 99.99 mass% or less, 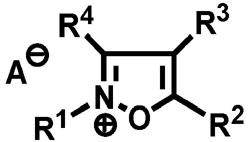 wherein A⁻ is ⁻OTf, R¹ and R² each represent a methyl group, R³ is -COOR⁵, R⁵ is a butyl group, and R⁴ is a hydrogen atom.
<59> An oxidizing method or a bleaching method including, bringing a treatment liquid into contact with an object, the treatment liquid being prepared from a liquid composition containing (A) an isoxazolium salt represented by the following general formula [hereinafter referred to as component (A)], (B) hydrogen peroxide [hereinafter referred as component (B)] and water, the liquid composition being acidic,
   wherein the treatment liquid has a pH of 5 or more and 10 or less,
   the treatment liquid contains component (A) in an amount of 0.0001 mass% or more and 1 mass% or less,
   the treatment liquid contains component (B) in an amount of 0.001 mass% or more and 3 mass% or less, and
   the treatment liquid contains water in an amount of 80 mass% or more and 99.99 mass% or less, 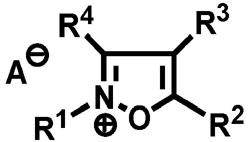 wherein A⁻ is ⁻OTf, R¹ and R² each represent a methyl group, R³ is -COOR⁵, R⁵ is a dodecyl group, and R⁴ is a hydrogen atom.
<60> An oxidizing method or a bleaching method including, bringing a treatment liquid into contact with an object, the treatment liquid being prepared from a liquid composition containing (A) an isoxazolium salt represented by the following general formula [hereinafter referred to as component (A)], (B) hydrogen peroxide [hereinafter referred as component (B)] and water, the liquid composition being acidic,
   wherein the treatment liquid has a pH of 5 or more and 10 or less,
   the treatment liquid contains component (A) in an amount of 0.0001 mass% or more and 1 mass% or less,
   the treatment liquid contains component (B) in an amount of 0.001 mass% or more and 3 mass% or less, and
   the treatment liquid contains water in an amount of 80 mass% or more and 99.99 mass% or less, 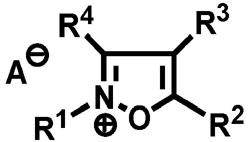 wherein A⁻ is ⁻OTf, R¹ and R² each represent a methyl group, R³ is -CONHR⁶, R⁶ is a dodecyl group, and R⁴ is a hydrogen atom.
<61> An oxidizing method or a bleaching method including, bringing a treatment liquid into contact with an object, the treatment liquid being prepared from a liquid composition containing (A) an isoxazolium salt represented by the following general formula [hereinafter referred to as component (A)], (B) hydrogen peroxide [hereinafter referred as component (B)] and water, the liquid composition being acidic,
   wherein the treatment liquid has a pH of 5 or more and 10 or less,
   the treatment liquid contains component (A) in an amount of 0.0001 mass% or more and 1 mass% or less,
   the treatment liquid contains component (B) in an amount of 0.001 mass% or more and 3 mass% or less, and
   the treatment liquid contains water in an amount of 80 mass% or more and 99.99 mass% or less, 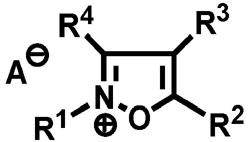 wherein A⁻ is ClO₄⁻, R¹ is a tert-butyl group, R² is a methyl group, and R³ and R⁴ each represent a hydrogen atom.
<62> A treatment liquid for oxidation or bleaching prepared from a liquid composition containing (A) an isoxazolium salt represented by the following general formula [hereinafter referred to as component (A)], (B) hydrogen peroxide [hereinafter referred as component (B)] and water, the liquid composition being acidic,
   wherein the treatment liquid has a pH of 5 or more and 10 or less at 25°C,
   the treatment liquid contains component (A) in an amount of 0.0001 mass% or more and 1 mass% or less,
   the treatment liquid contains component (B) in an amount of 0.001 mass% or more and 3 mass% or less, and
   the treatment liquid contains water in an amount of 80 mass% or more and 99.99 mass% or less, 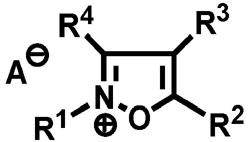 wherein A⁻ is ⁻OTf, R¹ and R² each represent a methyl group, R³ is -COOR⁵, R⁵ is a butyl group, and R⁴ is a hydrogen atom.
<63> A treatment liquid for oxidation or bleaching prepared from a liquid composition containing (A) an isoxazolium salt represented by the following general formula [hereinafter referred to as component (A)], (B) hydrogen peroxide [hereinafter referred as component (B)] and water, the liquid composition being acidic,
   wherein the treatment liquid has a pH of 5 or more and 10 or less at 25°C,
   the treatment liquid contains component (A) in an amount of 0.0001 mass% or more and 1 mass% or less,
   the treatment liquid contains component (B) in an amount of 0.001 mass% or more and 3 mass% or less, and
   the treatment liquid contains water in an amount of 80 mass% or more and 99.99 mass% or less, 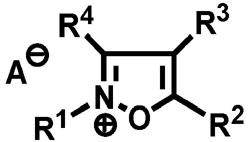 wherein A⁻ is ⁻OTf, R¹ and R² each represent a methyl group, R³ is -COOR⁵, R⁵ is a dodecyl group, and R⁴ is a hydrogen atom.
<64> A treatment liquid for oxidation or bleaching prepared from a liquid composition containing (A) an isoxazolium salt represented by the following general formula [hereinafter referred to as component (A)], (B) hydrogen peroxide [hereinafter referred as component (B)] and water, the liquid composition being acidic,
   wherein the treatment liquid has a pH of 5 or more and 10 or less at 25°C,
   the treatment liquid contains component (A) in an amount of 0.0001 mass% or more and 1 mass% or less,
   the treatment liquid contains component (B) in an amount of 0.001 mass% or more and 3 mass% or less, and
   the treatment liquid contains water in an amount of 80 mass% or more and 99.99 mass% or less, 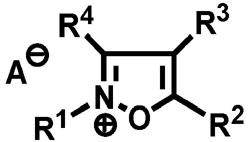 wherein A⁻ is ⁻OTf, R¹ and R² each represent a methyl group, R³ is -CONHR⁶, R⁶ is a dodecyl group, and R⁴ is a hydrogen atom.
<65> A treatment liquid for oxidation or bleaching prepared from a liquid composition containing (A) an isoxazolium salt represented by the following general formula [hereinafter referred to as component (A)], (B) hydrogen peroxide [hereinafter referred as component (B)] and water, the liquid composition being acidic,
   wherein the treatment liquid has a pH of 5 or more and 10 or less at 25°C,
   the treatment liquid contains component (A) in an amount of 0.0001 mass% or more and 1 mass% or less,
   the treatment liquid contains component (B) in an amount of 0.001 mass% or more and 3 mass% or less, and
   the treatment liquid contains water in an amount of 80 mass% or more and 99.99 mass% or less, 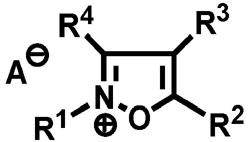 wherein A⁻ is ClO₄⁻, R¹ is a tert-butyl group, R² is a methyl group, and R³ and R⁴ each represent a hydrogen atom.
<66> A treatment liquid for oxidation or bleaching containing (A) an isoxazolium salt represented by the following general formula in an amount of 0.0001 mass% or more and 1 mass% or less, (B) hydrogen peroxide in an amount of 0.001 mass% or more and 3 mass% or less, and water in an amount of 80 mass% or more and 99.99 mass% or less, the treatment liquid having a pH of 5 or more and 10 or less at 25°C, 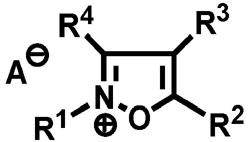 wherein A⁻ is ⁻OTf, R¹ and R² each represent a methyl group, R³ is -COOR⁵, R⁵ is a butyl group, and R⁴ is a hydrogen atom.
<67> A treatment liquid for oxidation or bleaching containing (A) an isoxazolium salt represented by the following general formula in an amount of 0.0001 mass% or more and 1 mass% or less, (B) hydrogen peroxide in an amount of 0.001 mass% or more and 3 mass% or less, and water in an amount of 80 mass% or more and 99.99 mass% or less, the treatment liquid having a pH of 5 or more and 10 or less at 25°C, 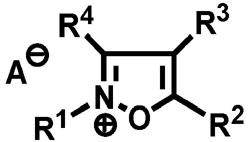 wherein A⁻ is ⁻OTf, R¹ and R² each represent a methyl group, R³ is -COOR⁵, R⁵ is a dodecyl group, and R⁴ is a hydrogen atom.
<68> A treatment liquid for oxidation or bleaching containing (A) an isoxazolium salt represented by the following general formula in an amount of 0.0001 mass% or more and 1 mass% or less, (B) hydrogen peroxide in an amount of 0.001 mass% or more and 3 mass% or less, and water in an amount of 80 mass% or more and 99.99 mass% or less, the treatment liquid having a pH of 5 or more and 10 or less at 25°C, 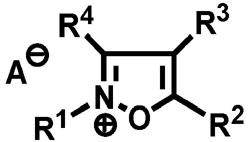 wherein A⁻ is ⁻OTf, R¹ and R² each represent a methyl group, R³ is -CONHR⁶, R⁶ is a dodecyl group, and R⁴ is a hydrogen atom.
<69> A treatment liquid for oxidation or bleaching containing (A) an isoxazolium salt represented by the following general formula in an amount of 0.0001 mass% or more and 1 mass% or less, (B) hydrogen peroxide in an amount of 0.001 mass% or more and 3 mass% or less, and water in an amount of 80 mass% or more and 99.99 mass% or less, the treatment liquid having a pH of 5 or more and 10 or less at 25°C, 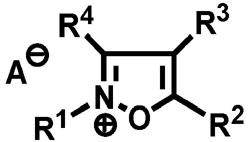 wherein A⁻ is ClO₄⁻, R¹ is a tert-butyl group, R² is a methyl group, and R³ and R⁴ each represent a hydrogen atom.
<70> A method for producing a treatment liquid for oxidation or bleaching using a liquid composition containing (A) an isoxazolium salt represented by the following general formula [hereinafter referred to as component (A)], (B) hydrogen peroxide [hereinafter referred as component (B)] and water, the liquid composition being acidic,
   wherein the treatment liquid has a pH of 5 or more and 10 or less at 25°C,
   the treatment liquid contains component (A) in an amount of 0.0001 mass% or more and 1 mass% or less,
   the treatment liquid contains component (B) in an amount of 0.001 mass% or more and 3 mass% or less, and
   the treatment liquid contains water in an amount of 80 mass% or more and 99.99 mass% or less,
   the method optionally including, mixing the liquid composition with water, 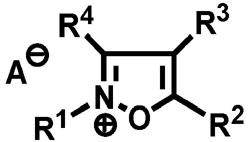 wherein A⁻ is ⁻OTf, R¹ and R² each represent a methyl group, R³ is -COOR⁵, R⁵ is a butyl group, and R⁴ is a hydrogen atom.
<71> A method for producing a treatment liquid for oxidation or bleaching using a liquid composition containing (A) an isoxazolium salt represented by the following general formula [hereinafter referred to as component (A)], (B) hydrogen peroxide [hereinafter referred as component (B)] and water, the liquid composition being acidic,
   wherein the treatment liquid has a pH of 5 or more and 10 or less at 25°C,
   the treatment liquid contains component (A) in an amount of 0.0001 mass% or more and 1 mass% or less,
   the treatment liquid contains component (B) in an amount of 0.001 mass% or more and 3 mass% or less, and
   the treatment liquid contains water in an amount of 80 mass% or more and 99.99 mass% or less,
   the method optionally including, mixing the liquid composition with water, 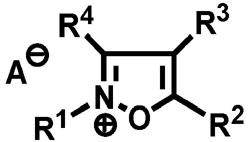 wherein A⁻ is ⁻OTf, R¹ and R² each represent a methyl group, R³ is -COOR⁵, R⁵ is a dodecyl group, and R⁴ is a hydrogen atom.
<72> A method for producing a treatment liquid for oxidation or bleaching using a liquid composition containing (A) an isoxazolium salt represented by the following general formula [hereinafter referred to as component (A)], (B) hydrogen peroxide [hereinafter referred as component (B)] and water, the liquid composition being acidic,
   wherein the treatment liquid has a pH of 5 or more and 10 or less at 25°C,
   the treatment liquid contains component (A) in an amount of 0.0001 mass% or more and 1 mass% or less,
   the treatment liquid contains component (B) in an amount of 0.001 mass% or more and 3 mass% or less, and
   the treatment liquid contains water in an amount of 80 mass% or more and 99.99 mass% or less,
   the method optionally including, mixing the liquid composition with water, 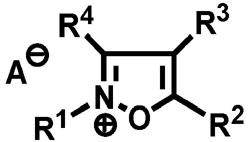 wherein A⁻ is ⁻OTf, R¹ and R² each represent a methyl group, R³ is -CONHR⁶, R⁶ is a dodecyl group, and R⁴ is a hydrogen atom.
<73> A method for producing a treatment liquid for oxidation or bleaching using a liquid composition containing (A) an isoxazolium salt represented by the following general formula [hereinafter referred to as component (A)], (B) hydrogen peroxide [hereinafter referred as component (B)] and water, the liquid composition being acidic,
   wherein the treatment liquid has a pH of 5 or more and 10 or less at 25°C,
   the treatment liquid contains component (A) in an amount of 0.0001 mass% or more and 1 mass% or less,
   the treatment liquid contains component (B) in an amount of 0.001 mass% or more and 3 mass% or less, and
   the treatment liquid contains water in an amount of 80 mass% or more and 99.99 mass% or less,
   the method optionally including, mixing the liquid composition with water, 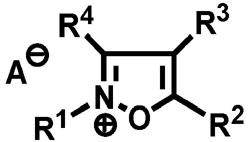 wherein A⁻ is ClO₄⁻, R¹ is a tert-butyl group, R² is a methyl group, and R³ and R⁴ each represent a hydrogen atom.

### Examples

The compounds produced in the following synthesis examples were used as evaluation compounds in (some) examples and reference examples. Each synthesis was performed using an eggplant flask.

### (1) Synthesis example 1

4-(butoxycarbonyl)-2,5-dimethylisoxazol-2-ium trifluoromethanesulfonate, the evaluation compound of example 2, was synthesized in the following two steps.

0.87 g (11.8 mmol, 1.5 eq.) of 1-butanol was added to a dichloromethane (15.74 mL) solution of 1 g (7.87 mmol) of 5-methylisoxazole-4-carboxylic acid, and this was cooled to 0°C. A catalytic amount of N,N-dimethyl-4-aminopyridine (DMAP), and further, 1.81 g of 1-(3-dimethylaminopropyl)-3-ethylcarbodiimide hydrochloride (EDCl) (9.44 mol, 1.2 eq.), were added thereto, and stirred at room temperature (25°C) for 1 hour. After the reaction ended, a saturated ammonium chloride aqueous solution was added to the reaction solution to stop the reaction, and extraction was carried out with dichloromethane. The obtained organic layer was washed with water and a saturated saline solution, and thereafter dried with anhydrous sodium sulfate and concentrated with a rotary evaporator to obtain a crude product. The obtained crude product was purified with silica gel column chromatography (hexane: ethyl acetate = 4:1 -> 1:1) to obtain butyl 5-methylisoxazole-4-carboxylate in an amount of 1.4 g (7.6 mmol, yield 97%). The scheme of this reaction is as follows:

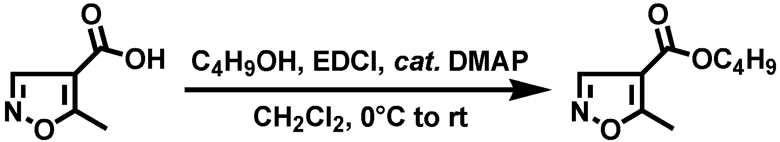

The ¹H NMR measurement results of butyl 5-methylisoxazole-4-caboxylate were as follows:
¹H NMR (400 MHz, CDCl₃) δ = 8.46 (1H, s), 4.27 (2H, t, J = 6.4 Hz), 2.70 (3H, s), 1.73-1.68 (2H, m), 1.47-1.41 (2H, m), 0.97 (3H, t, J = 7.2 Hz) ppm.
Note that the ¹H NMR measurement conditions were as shown below (the same applies hereinafter).

### <¹H NMR measurement conditions>

· Device: VNMR 400MR DD2 (manufactured by Agilent Technologies, Inc.)
· Measurement temperature: 25°C
· Waiting time: 10 seconds
· Number of times of integration: 8 times
· Observation range: 6410.3 Hz
· Pulse: 45°
· Data point: 65536

A dichloromethane (10.92 mL) solution of 1 g (5.46 mmol) of butyl 5-methylisoxazole-4-carboxylate was cooled to 0°C. 0.99 g (6.00 mol, 1.1 eq.) of methyl trifluoromethanesulfonate was added thereto, and stirred at room temperature (25°C) for 1 hour. After the reaction ended, the reaction solution was concentrated with a rotary evaporator to obtain a crude product. The obtained crude product was washed with hexane to obtain 4-(butoxycarbonyl)-2,5-dimethylisoxazol-2-ium trifluoromethanesulfonate in an amount of 1.9 g (5.46 mmol, yield quant.) The scheme of this reaction is as follows:

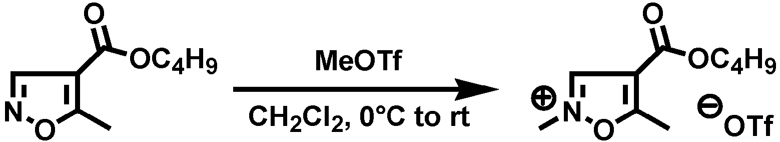

The ¹H NMR measurement results of 4-(butoxycarbonyl)-2,5-dimethylisoxazol-2-ium trifluoromethanesulfonate were as follows:
¹H NMR (400 MHz, CDCl₃) δ = 9.8 (1H, s), 4.51 (3H, s), 4.35 (2H, t, J = 7.2 Hz), 2.92 (3H, s), 1.77-1.70 (2H, m), 1.48-1.39 (2H, m), 0.96 (3H, t, J = 7.2 Hz) ppm.

### (2) Synthesis example 2

4-((dodecyloxy)carbonyl)-2,5-dimethylisoxazol-2-ium trifluoromethanesulfonate, the evaluation compound of example 3, was synthesized in the following two steps.

2.2 g (11.8 mmol, 1.5 eq.) of lauryl alcohol was added to a dichloromethane (15.74 mL) solution of 1 g (7.87 mmol) of 5-methylisoxazole-4-carboxylic acid, and this was cooled to 0°C. A catalytic amount of DMAP and further 1.81 g of EDCl (9.44 mol, 1.2 eq.) were added thereto, and stirred at room temperature (25°C) for 1 hour. After the reaction ended, a saturated ammonium chloride aqueous solution was added to the reaction solution to stop the reaction, and extraction was carried out with dichloromethane. The obtained organic layer was washed with water and a saturated saline solution, and thereafter dried with anhydrous sodium sulfate and concentrated with a rotary evaporator to obtain a crude product. The obtained crude product was purified with silica gel column chromatography (hexane: ethyl acetate = 4:1 -> 1:1) to obtain dodecyl 5-methylisoxazole-4-carboxylate in an amount of 2.2 g (7.45 mmol, yield 95%). The scheme of this reaction is as follows:

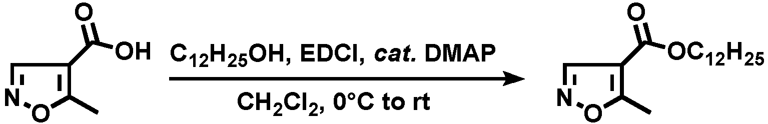

The ¹H NMR measurement results of dodecyl 5-methylisoxazole-4-carboxylate were as follows:
¹H NMR (400 MHz, CDCl₃) δ = 8.47 (1H, s), 4.26 (2H, t, J = 6.8 Hz), 2.70 (3H, s), 1.74-1.71 (2H, m), 1.40-1.26 (18H, m), 0.88 (3H, t, J = 6.8 Hz) ppm.

A dichloromethane (6.77 mL) solution of 1 g (3.39 mmol) of dodecyl 5-methylisoxazole-4-carboxylate was cooled to 0°C. 0.61 g (3.72 mol, 1.1 eq.) of methyl trifluoromethanesulfonate was added thereto, and stirred at room temperature (25°C) for 1 hour. After the reaction ended, the reaction solution was concentrated with a rotary evaporator to obtain a crude product. The obtained crude product was washed with hexane to obtain 4-((dodecyloxy)carbonyl)-2,5-dimethylisoxazol-2-ium trifluoromethanesulfonate in an amount of 1.56 g (3.39 mmol, yield quant.) The scheme of this reaction is as follows:

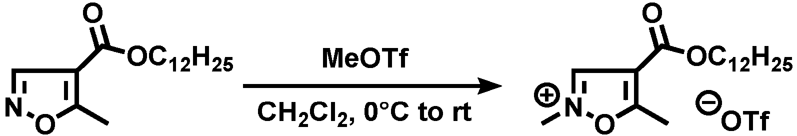

The ¹H NMR measurement results of 4-((dodecyloxy)carbonyl)-2,5-dimethylisoxazol-2-ium trifluoromethanesulfonate were as follows:
¹H NMR (400 MHz, CDCl₃) δ = 9.88 (1H, s), 4.53 (3H, s), 4.34 (2H, t, J = 6.8 Hz), 2.93 (3H, s), 1.78-1.71 (2H, m), 1.39-1.26 (18H, m), 0.88 (3H, t, J = 6.8 Hz) ppm.

### (2) Synthesis example 3

4-(dodecylcarbamoyl)-2,5-dimethylisoxazol-2-ium trifluoromethanesulfonate, the evaluation compound of example 4, was synthesized in the following two steps.

2.19 g (11.8 mmol, 1.5 eq.) of lauryl amine was added to a dichloromethane (15.74 mL) solution of 1 g (7.87 mmol) of 5-methylisoxazole-4-carboxylate, and this was cooled to 0°C.

A catalytic amount of DMAP and further 1.81 g of EDCl (9.44 mol, 1.2 eq.) were added thereto, and stirred at room temperature (25°C) for 1 hour. After the reaction ended, a saturated ammonium chloride aqueous solution was added to the reaction solution to stop the reaction, and extraction was carried out with dichloromethane. The obtained organic layer was washed with water and a saturated saline solution, and thereafter dried with anhydrous sodium sulfate and concentrated with a rotary evaporator to obtain a crude product. The obtained crude product was purified with silica gel column chromatography (hexane: ethyl acetate = 4:1 -> 1:1) to obtain N-dodecyl-5-methylisoxazole-4-carboxamide in an amount of 2.2 g (7.45 mmol, yield 95%). The scheme of this reaction is as follows:

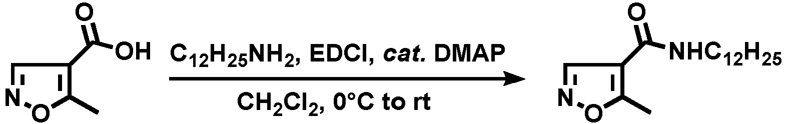

The ¹H NMR measurement results of N-dodecyl-5-methylisoxazole-4-carboxamide were as follows:
¹H NMR (400 MHz, CDCl₃) δ = 8.32 (1H, s), 5.77 (1H, s), 3.38 (2H, t, J = 6 Hz), 2.71 (3H, s), 1.63-1.55 (2H, m), 1.33-1.26 (18H, m), 0.88 (3H, t, J = 6.8 Hz) ppm.

A dichloromethane (6.79 mL) solution of 1 g (3.40 mmol) of N-dodecyl-5-methylisoxazole-4-carboxamide was cooled to 0°C. 0.61 g (3.72 mol, 1.1 eq.) of methyl trifluoromethanesulfonate was added thereto, and stirred at room temperature (25°C) for 1 hour. After the reaction ended, the reaction solution was concentrated with a rotary evaporator to obtain a crude product. The obtained crude product was washed with hexane to obtain 4-(dodecylcarbamoyl)-2,5-dimethylisoxazol-2-ium trifluoromethanesulfonate in an amount of 1.56 g (3.39 mmol, yield quant.) The scheme of this reaction is as follows:

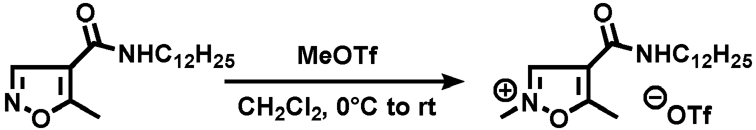

The ¹H NMR measurement results of 4-(dodecylcarbamoyl)-2,5-dimethylisoxazol-2-ium trifluoromethanesulfonate were as follows:
¹H NMR (400 MHz, CDCl₃) δ = 10.10 (1H, s), 7.92 (1H, s), 4.44 (3H, s), 3.37-3.32 (2H, m), 2.94 (3H, s), 1.62-1.55 (2H, m), 1.30-1.25 (18H, m), 0.88 (3H, t, J = 6.8 Hz) ppm.

Note that the evaluation compound of example 1 was the following compound (N-tert-butyl-5-methylisoxazolium perchlorate: manufactured by Tokyo Chemical Industry Co., Ltd.) :

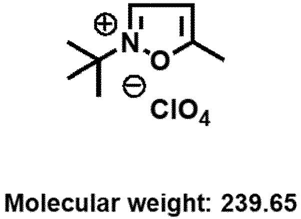

### <Reagent>

The reagents below were used in examples and reference examples.
· Phosphoric acid (85%): manufactured by FUJIFILM Wako Pure Chemical Corporation
· Disodium hydrogen phosphate: manufactured by FUJIFILM Wako Pure Chemical Corporation
· Potassium dihydrogen phosphate: manufactured by FUJIFILM Wako Pure Chemical Corporation
· Sodium 3-(trimethylsilyl)-1-propane-1,1,2,2,3,3-d6-sulfonate: manufactured by FUJIFILM Wako Pure Chemical Corporation
· Ethanol (99.5%): manufactured by FUJIFILM Wako Pure Chemical Corporation
· Curcumin: manufactured by FUJIFILM Wako Pure Chemical Corporation
· Acetonitrile: manufactured by FUJIFILM Wako Pure Chemical Corporation
· Hydrogen peroxide (30%): manufactured by FUJIFILM Wako Pure Chemical Corporation

Those shown in parenthesis are effective amounts. The amounts in the tables are expressed in terms of effective amounts.

### [Example]

### <Evaluation of storage stability>

A phosphate buffer solution with pH = 2.5 was prepared using phosphoric acid, disodium hydrogen phosphate and heavy water, and a solution of 3% of hydrogen peroxide of component (B), 26.4 mM of component (A) in Table 1 and 3 mM of sodium 3-(trimethylsilyl)-1-propane-1,1,2,2,3,3-d6-sulfonate (standard substance) was prepared using this. This solution corresponds to a liquid composition whose composition excluding the above standard substance is composition (mass%) in Table 1. The liquid compositions of the examples in Table 1 can be used as oxidizing agents or bleaching agents. Immediately after prepared, the liquid composition was subjected to ¹H NMR measurement, and stored at room temperature (25°C) after the measurement. After a lapse of 1 hour from the first measurement, ¹H NMR measurement was carried out once again. The ¹H NMR measurement conditions were the same as above. A reduction amount in the peaks of the evaluation compound (component (A)) was calculated from the peak ratios to the standard substance in an obtained plot, and a value of remaining rate (%) of component (A) was determined by the internal standard method to evaluate storage stability. The results are shown in Table 1. A higher numerical value means more excellent storage stability.

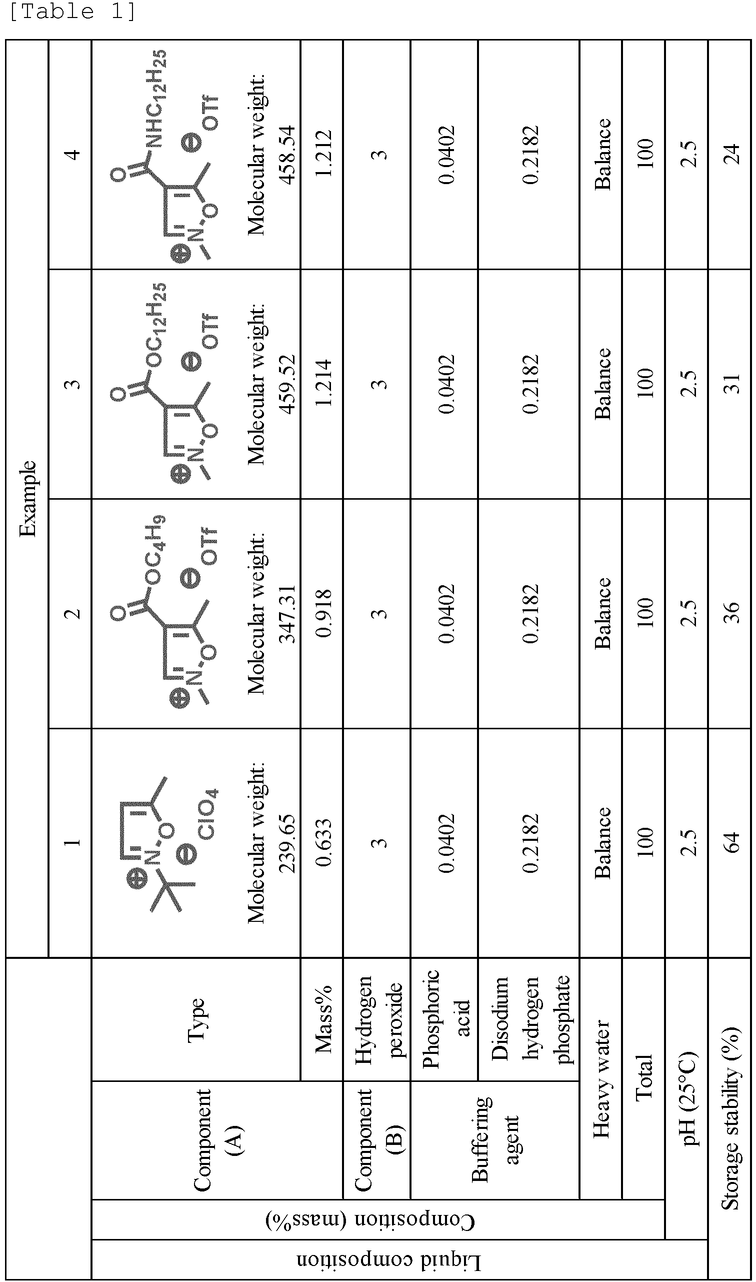

### [Reference example]

### <Evaluation of oxidizing power>

A solution of 5.29 mM of component (A) (Table 2) (which is referred to as solution A) was prepared using ion-exchanged water, a solution of 1765 mM of component (B) (which is referred to as solution B) was prepared using ion-exchanged water and 30% hydrogen peroxide, and a solution of 1.32 mM of curcumin was prepared using ethanol. 0.1 mL of solution B and 1 mL of solution A were added to 18.8 mL of a phosphate buffer solution adjusted to have pH = 7.3 using potassium dihydrogen phosphate and disodium hydrogen phosphate, and stirred for 10 seconds, and then, 0.1 mL of the curcumin solution was added thereto and stirred for 10 minutes (the final concentrations of component (A), component (B) and curcumin were 0.264 mM, 8.824 mM and 0.0066 mM, respectively). 10 minutes later, 0.5 mL of the reaction solution was taken, and 0.5 mL of a quenching liquid (0.5 M aqueous phosphate solution/acetonitrile = 1:1 (vol)) was added thereto to obtain a sample solution. This was quantified with HPLC to determine a remaining amount of curcumin. Note that this evaluation corresponds to a test for evaluating oxidative decomposition activity when the liquid compositions in Table 1 are used under a neutral condition. A higher numerical value means more excellent oxidative decomposition activity.

As a result of the quantification with HPLC, the oxidative decomposition activity (oxidizing power (%) calculated by the formula below) of the liquid compositions in Table 2 under the neutral condition was as shown in Table 2. Note that the HPLC conditions were as shown below. Oxidizing power (%) = 100 × [(use amount of curcumin - remaining amount of curcumin)/use amount of curcumin]

### <HPLC conditions>

- Device
   SHIMADZU CBM-20A
   Pump: LC-20A, UV-Vis detector: SPD-20A, PDA detector: SPD-M20A, Column oven: CTO-20A, Auto sampler: SIL-20A
- Analysis conditions
   Column: L-column ODS, 150 mm × 4.6 mm, 5 µm (Chemicals Evaluation and Research Institute, Japan), Oven
   temperature: 40°C, Detector: UV (430 nm), Injection
   volume: 10 µl, Flow rate: 1.0 mL/min, Mobile phase: A = 50 mM phosphoric acid, B = acetonitrile

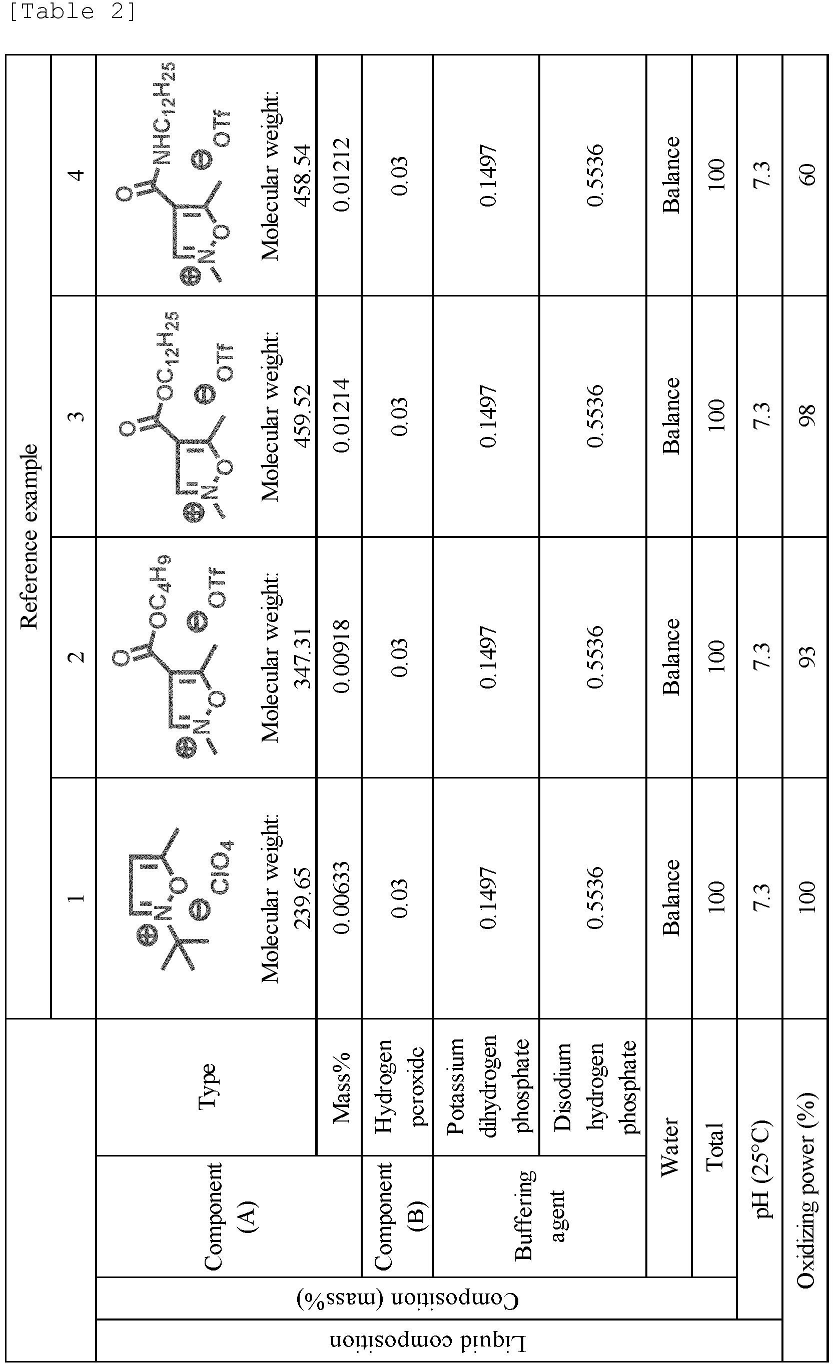

## Claims

1. A liquid composition comprising (A) an isoxazolium salt [hereinafter referred to as component (A)] and water, the composition being acidic.

2. The liquid composition according to claim 1, wherein the component (A) is a compound represented by the following general formula: 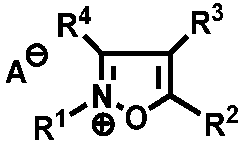 wherein A⁻ represents an anion, R¹ and R² each independently represent an alkyl group with 1 or more and 24 or less carbons which may contain a heteroatom, R³ and R⁴ each independently represent a group selected from a hydrogen atom, an alkyl group with 1 or more and 24 or less carbons which may contain a heteroatom, -COOR⁵ and - CONHR⁶, and R⁵ and R⁶ each represent an alkyl group with 2 or more and 23 or less carbons.

3. The liquid composition according to claim 1 or 2, further comprising (B) hydrogen peroxide [hereinafter referred to as component (B)].

4. The liquid composition according to claim 3, wherein a content of the component (A) relative to a content of 1 part by mole of the component (B) is 1 part by mole or more and 100 parts by mole or less.

5. The liquid composition according to any of claims 1 to 4, wherein the composition has a pH of less than 7 at 25°C.

6. The liquid composition according to any of claims 1 to 5, wherein the composition is used for oxidizing agents.

7. The liquid composition according to any of claims 1 to 5, wherein the composition is used for bleaching agents.

8. A method for storing an isoxazolium salt comprising, storing (A) an isoxazolium salt [hereinafter referred to as component (A)] in an acidic solution.

9. The storing method according to claim 8, wherein the component (A) is stored under the coexistence of hydrogen peroxide.

10. An oxidizing method comprising, bringing a treatment liquid prepared from the liquid composition according to any of claims 1 to 7 into contact with an object in the presence of hydrogen peroxide.

11. A bleaching method comprising, bringing a treatment liquid prepared from the liquid composition according to any of claims 1 to 7 into contact with an object in the presence of hydrogen peroxide.

12. Use of the liquid composition according to any of claims 1 to 7 for the production of oxidizing agents.

13. Use of the liquid composition according to any of claims 1 to 7 for the production of bleaching agents.
